# EUROPEAN PATENT SPECIFICATION

(11) **EP 2 375 997 B1**
(45) Date of publication and mention of the grant of the patent: **19.06.2013**
(21) Application number: 09775475.8
(22) Date of filing: 15.12.2009
(51) Int. Cl.: A61B 17/064, A61B 17/068

(54) **VARIABLE THICKNESS TACKING DEVICES**
HEFTVORRICHTUNGEN MIT VARIABLER DICKE
DISPOSITIFS DE MAINTIEN EN PLACE POUR ÉPAISSEURS VARIABLES

(30) Priority: 19.12.2008 US 139148 P
(43) Date of publication of application: 19.10.2011
(73) Proprietor: Cook Medical Technologies LLC, Bloomington, IN 47404 (US)
(72) Inventor: KARPIEL, John, A., Winston-Salem NC 27106 (US); MCLAWHORN, Tyler, E., Winston-Salem NC 27106 (US); AGUIRRE, Andres, F., Burlington NC 27215 (US)
(74) Representative: Garratt, Peter Douglas
(86) International application number: PCT/US2009/067994
(87) International publication number: WO 2010/080387

(56) References cited:
- US-A1- 2001 037 130
- US-A1- 2001 039 435
- US-A1- 2005 038 370
- US-A1- 2007 129 755

## Description

### BACKGROUND

The present invention relates generally to medical devices, and more particularly, to devices for engaging tissue or facilitating closure of a bodily opening.

Perforations in tissue or bodily walls may be formed intentionally or unintentionally. For example, an unintentional ventral abdominal hernia may be formed in the abdominal wall due to heavy lifting, coughing, strain imposed during a bowel movement or urination, fluid in the abdominal cavity, or other reasons.

Intentional perforations may be formed, for example, during surgical procedures such as translumenal procedures. In a translumenal procedure, one or more instrument, such as an endoscope, may be inserted through a visceral wall, such as the stomach wall. During a translumenal procedure, a closure instrument may be used to close the perforation in the visceral wall. Depending on the structure comprising the perforation, it may be difficult to adequately close the perforation and prevent leakage of bodily fluids.

Attempts to seal perforations have been performed by coupling a graft member to tissue. For example, a graft material such as a mesh or patch may be disposed to overlap with tissue surrounding the perforation. The graft material then may be secured to the surrounding tissue in an attempt to effectively cover and seal the perforation. In order to secure the graft material to the surrounding tissue, sutures commonly are manually threaded through the full thickness of the surrounding tissue, then tied down and knotted. However, such manual suturing techniques may be time consuming and/or difficult to perform. Moreover, when closing intentional openings formed during translumenal procedures, suturing techniques may permit leakage of bodily fluids, and may be unreliable and difficult to reproduce.

Further attempts to seal intentional or unintentional openings in tissue have been performed using mechanical devices such as clips, tacks, staples, and fasteners. Such devices may be delivered towards a target tissue site and deployed to engage tissue surrounding the opening. However, typically such mechanical devices cannot readily accommodate unexpected localized variations in tissue and graft thickness, or cannot make an adjustment after an improper estimation of tissue and graft thickness. If the mechanical devices cannot accommodate such variations in tissue or graft thickness, it may result in an improper deployment of the device or cause gap formations and potential leakage.

With reference to figures 31, US20070129755 discloses a clip for fixing tissue of finite thickness and comprising a spring arranged to pull opposed tissue engaging members towards each other. US20010037130 discloses compressive tissue tacks whose effective length may be varied.

### SUMMARY

The present invention is a tacking device for engaging tissue, as set out in appended claims 1 and 2, which may be useful for coupling a graft member to tissue or facilitating closure of a bodily opening. In one embodiment, the tacking device comprises a main body having proximal and distal ends, a proximal base member disposed at the proximal end of the main body, and at least one tissue engaging member disposed at the distal end of the main body. A spring member, which surrounds the main body, has a proximal end that contacts the proximal base member.

In use, the spring member has a relaxed state in which it is biased to extend distally towards the at least one tissue engaging member, and further has a compressed state in which the distal end of the spring member is spaced further apart from the at least one tissue engaging member. Therefore, tissues and/or graft members of varying thicknesses may be captured between the distal end of the spring and the tissue engaging member.

Advantageously, the provision of the spring member may facilitate coupling of a graft member to tissue, regardless of a thickness of the tissue and a thickness of the graft member. Since the spring member is biased to the relaxed state, it can capture and provide a compressive force upon any combined thickness of the tissue and the graft member, and can accommodate localized variations in thickness of the tissue and/or the graft member without resulting in leakage.

A delivery system for deploying the tacking device of the present invention may comprise an outer sheath and a catheter, each having a lumen. The catheter is configured for longitudinal movement within the lumen of the outer sheath, and the tacking device is configured to be selectively advanced through the lumen of the catheter. Preferably, at least one wedge member is disposed along a flexible distal region of the catheter. The wedge member is configured to form a constriction at a distal end of the catheter when the outer sheath is positioned over the distal end of the catheter. Distal advancement of the tacking device relative to the constriction is configured to cause a distal base member of the tacking device to engage the constriction, and further configured to cause the tissue engaging member to extend distally beyond the distal end of the catheter to engage tissue. At this time, the spring member may be held in the compressed state near the distal end of the catheter. Subsequent proximal retraction of the outer sheath, beyond the distal end of the catheter and the wedge member, permits radially outward movement of the distal end of the catheter and the wedge member to thereby remove the constriction and permit deployment of the entire tacking device from the distal end of the catheter.

### BRIEF DESCRIPTION OF THE DRAWINGS

The invention can be better understood with reference to the following drawings and description. The components in the figures are not necessarily to scale, emphasis instead being placed upon illustrating the principles of the invention. Moreover, in the figures, like referenced numerals designate corresponding parts throughout the different views.

FIG. 1 is a side view of a tacking device of a first embodiment in a relaxed state.

FIG. 2 is a side view of the tacking device of FIG. 1 in a compressed state.

FIGS. 3-5 are side-sectional views illustrating an exemplary delivery system and sequence of deployment for at least one tacking device provided in accordance with FIGS. 1-2.

FIG. 6 illustrates one exemplary use of multiple tacking devices of FIGS. 1-2 to couple a graft member to tissue to treat a ventral abdominal hernia.

FIG. 7 is a perspective view illustrating features of a distal region of a catheter of a delivery system.

FIGS. 8-9 are side-sectional views of an alternative embodiment of a delivery system.

FIG. 10 is a side view of a tacking device of an alternative embodiment in a relaxed state.

FIG. 11 is a side view of the tacking device of FIG. 10 in a compressed state.

FIGS. 12-13 are side-sectional views illustrating an exemplary delivery system and partial sequence of deployment of tacking devices provided in accordance with FIGS. 10-11.

### DETAILED DESCRIPTION OF THE PREFERRED EMBODIMENTS

In the present application, the term "proximal" refers to a direction that is generally towards a physician during a medical procedure, while the term "distal" refers to a direction that is generally towards a target site within a patient's anatomy during a medical procedure.

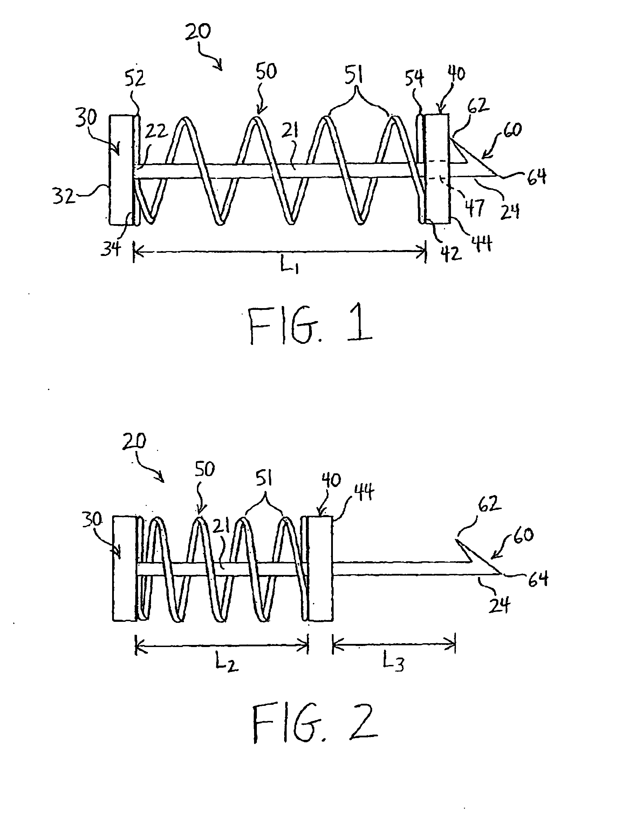

Referring now to FIG. 1, a first embodiment of a tacking device 20 is shown. In this embodiment, the tacking device 20 comprises a main body 21 having a proximal end 22 and a distal end 24. The tacking device 20 further comprises a proximal base member 30 having proximal and distal surfaces 32 and 34. Optionally, the tacking device 20 may comprise a distal base member 40 having proximal and distal surfaces 42 and 44, as shown in FIG. 1. The distal base member 40 has an aperture 47 formed therein, which may comprise an inner diameter that is slightly larger than an outer diameter of the main body 21. The main body 21, along with the proximal and distal base members 30 and 40, may be formed from any suitable material including, but not limited to, biocompatible plastics, stainless steel and/or shape-memory alloys.

The tacking device 20 further comprises a spring member 50 having a proximal end 52 and a distal end 54. The spring member 50 circumferentially surrounds at least a portion of the main body 21. In the embodiment of FIGS. 1-2, the spring member 50 is disposed between the proximal and distal base members 30 and 40. In particular, the proximal end 52 of the spring member 50 contacts the distal surface 34 of the proximal base member 30, while the distal end 54 of the spring member 50 contacts the proximal surface 42 of the distal base member 40. The spring member 50 may be secured to the proximal and distal base members 30 and 40 using an adhesive, solder, weld, mechanical attachment device, or any other suitable mechanism. Alternatively, the spring member 50 may be disposed in an abutting relationship with the proximal and distal base members 30 and 40.

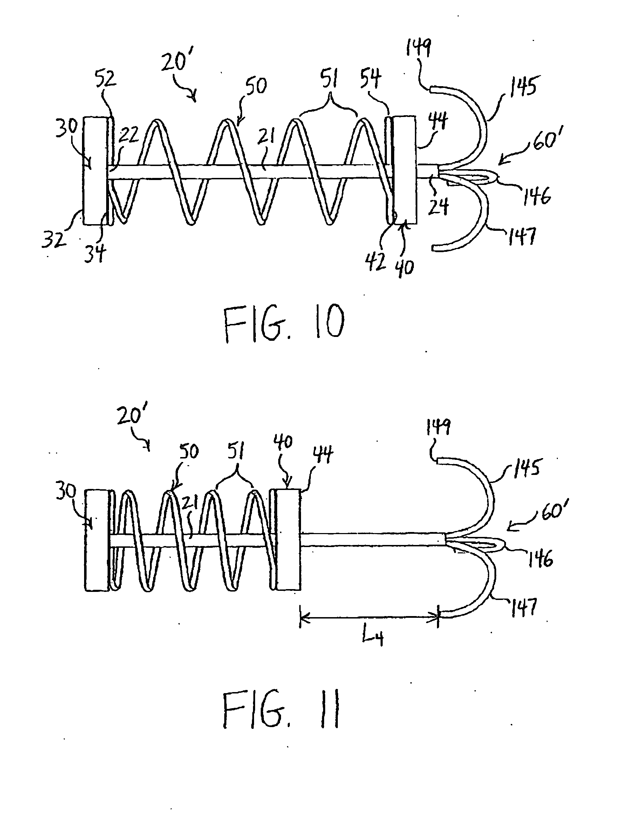

At least one tissue engaging member 60 is disposed at the distal end 24 of the main body 21. The tissue engaging member 60 may comprise any suitable shape and configuration for piercing, abutting, or anchoring into tissue. In the example of FIGS. 1-2, the tissue engaging member 60 comprises a single, substantially rigid member having a proximal edge 62 and a distal edge 64, forming a sharpened, hook-shaped tip therebetween. However, as will be explained below with respect to FIGS. 10-13, the tissue engaging member 60 alternatively may comprise one or more deployable members having contracted and expanded states, wherein the deployable members are configured to engage tissue in the expanded states.

The spring member 50 comprises relaxed and compressed states, depicted in FIGS. 1-2, respectively. The spring member 50 comprises a first length L₁ in the relaxed state, as shown in FIG. 1. In the relaxed state, the spring member 50 is longitudinally expanded and the distal end 54 preferably is disposed abutting the tissue engaging member 60. In the embodiment shown, in which the optional distal base member 40 is attached to the distal end 54 of the spring member 50, the distal surface 44 of the distal base member 40 is disposed in an abutting relationship with the proximal edge 62 of the tissue engaging member 60. Accordingly, one or more segments of tissue or graft material having varying thicknesses, no matter how thin, may be captured between the distal surface 44 of the distal base member 40 and the tissue engaging member 60 when the spring member 50 is biased towards the relaxed state, as explained in further detail below.

The spring member 50 further comprises a second length **L₂** in the compressed state, as shown in FIG. 2. The second length L₂ is less than the first length **L₁** due to compression of the spring member 50, and therefore, the distal end 54 of the spring member 50 and the distal base member 40 are spaced further apart from the tissue engaging member 60. A spacing **L₃** is formed between the distal surface 44 of the distal base member 40 and the proximal edge 62 of the tissue engaging member 60, as shown in FIG. 2. As will be apparent, while the distal base member 40 is depicted as approximately halfway between the proximal base member 30 and the tissue engaging member 60 in FIG. 2, the distal base member 40 may be positioned closer to or further from the proximal base member 30 when the spring member 50 is in a compressed state. In this state, one or more segments of tissue or graft material may be positioned between the distal base member 40 and the tissue engaging member 60, as explained in further detail below.

The spring member 50 may comprise any suitable material, such as stainless steel. Further, the spring member 50 may comprise a shape and configuration that may be tailored based on a given application. In particular, the diameter, wire thickness, stiffness and/or other features of the spring member 50 may be varied as needed for a particular procedure to meet anatomical constraints and/or vary the force imposed on tissue segments.

In the embodiment of FIGS. 1-2, the proximal and distal base members 30 and 40 comprise generally cylindrical shapes, which may facilitate insertion through a lumen 78 of a catheter 70, as explained further below. However, the proximal and distal base members 30 and 40 alternatively may comprise different shapes. Further, as will be explained further below, the distal base member 40 preferably comprises an outer diameter sized to selectively engage a constriction 79 of the catheter 70, but the proximal base member 30 and the spring member 50 may comprise reduced diameter profiles relative to the distal base member 40.

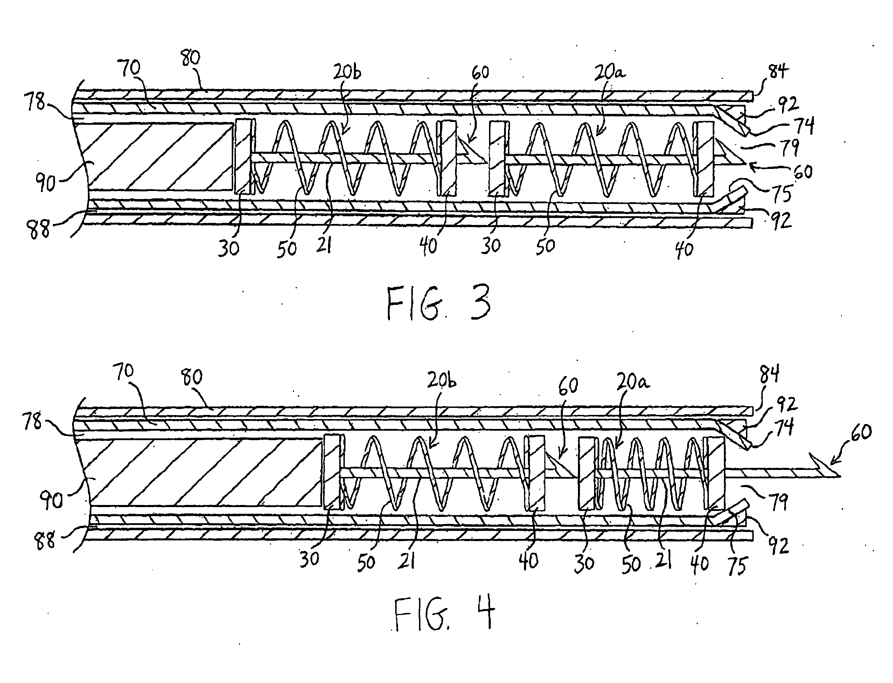

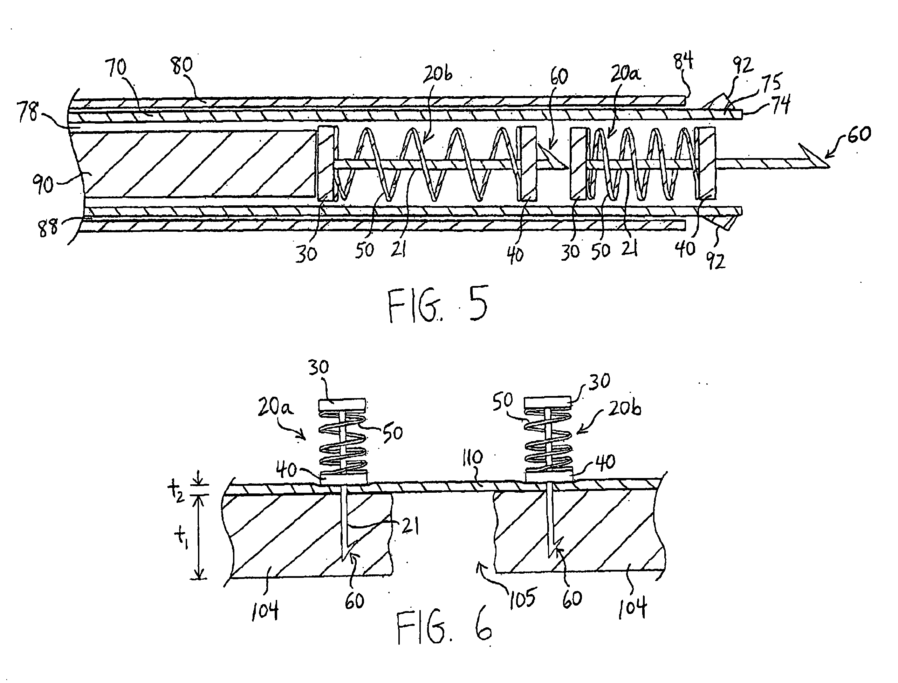

Referring now to FIGS. 3-5, an exemplary delivery system is described for delivery and deployment of at least one of the tacking devices 20 of FIGS. 1-2. In the embodiment of FIGS. 3-5, first and second tacking devices 20a and 20b are provided for sequential deployment. The first and second tacking devices 20a and 20b may be used to facilitate treatment of a perforation 105, such as a ventral hernia located in tissue 104 of the abdominal wall, using a graft member 110, as explained in FIG. 6 below.

In FIG. 3, the delivery system comprises a catheter 70 having a lumen 78, and further comprises an outer sheath 80 having a lumen 88. The catheter 70 comprises an outer diameter that is less than an inner diameter of the outer sheath 80, thereby allowing the catheter 70 to be longitudinally advanced within the lumen 88 of the outer sheath 80. The catheter 70 further comprises an inner diameter that is generally larger than an outer diameter of the first and second tacking devices 20a and 20b, thereby allowing the first and second tacking devices 20a and 20b to be loaded within the lumen 78 of the catheter 70, as shown in FIG. 3.

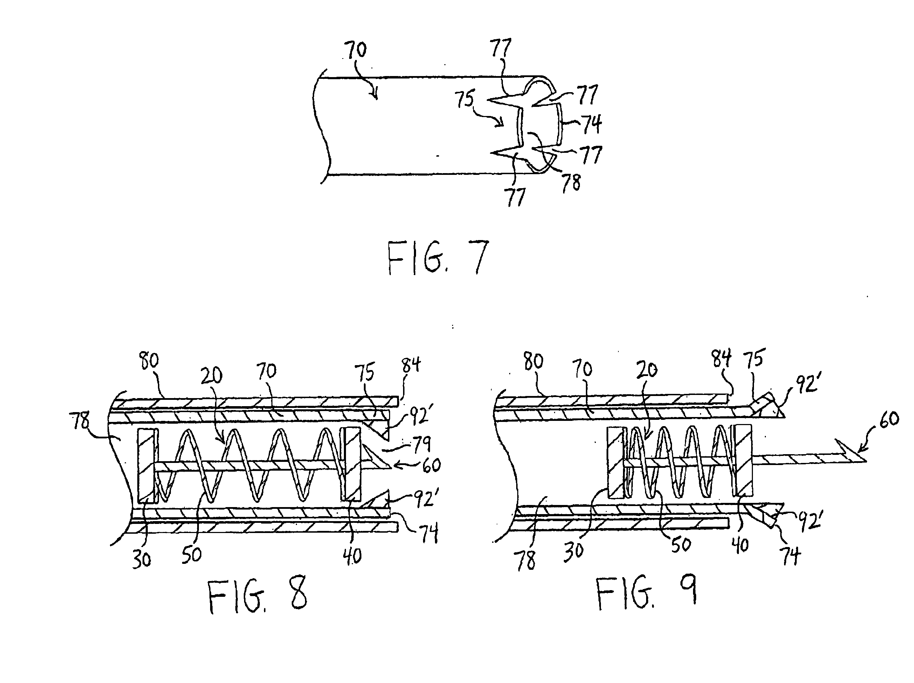

The catheter 70 comprises a distal end 74 and a flexible distal region 75. The flexible distal region 75 may be selectively moved in radially inward and outward directions, for purposes described further below. Preferably, a plurality of slits 77 are formed in the distal end 74, as shown in FIG. 7, to permit the radial flexibility along the distal region 75.

At least one wedge member 92 may be used to form a constriction 79 at the distal end 74 of the catheter 70. In the embodiment of FIGS. 3-5, the at least one wedge member 92 has a triangular shape arc is disposed between the catheter 70 and the outer sheath 80, causing the flexible distal region 75 of the catheter 70 to move radially inward to form the constriction 79, as shown in FIGS. 3-4. The wedge member 92 may comprise a biocompatible glue, plastic, metal or other suitable material, and may comprise other shapes besides the triangular shape depicted to accomplish the objectives described below. Alternatively, one or more wedge members 92 may be formed as an integral portion of the catheter 70 at the distal region 75.

The outer sheath 80 may comprise a rigid or substantially rigid material, such as stainless steel or plastic materials, which substantially prohibits radial outward movement of the wedge member 92 and the flexible distal region 75 of the catheter 70, when a distal end 84 of the outer sheath 80 covers these regions, as shown in FIGS. 3-4. However, when the distal end 84 of the outer sheath 80 is retracted proximally beyond the wedge member 92 and the flexible distal region 75 of the catheter 70, the flexible distal region 75 may move radially outward and the constriction 79 may be removed, as depicted in FIG. 5 below.

In one exemplary method to treat the perforation 105 of FIG. 6 using the graft member 110, the first and second tacking devices 20a and 20b may be loaded sequentially such that the first tacking device 20a is loaded distal to the second tacking device 20b within the lumen 78 of the catheter 70, as shown in FIG. 3. A stylet 90 may be positioned in the lumen 78 at a location proximal to the second tacking device 20b. It should be noted that while two tacking devices are shown in this example, any number may be used and sequentially loaded into the catheter 70.

The outer sheath 80 is positioned over the catheter 70 such that the constriction 79 is formed via the wedge member 92, as shown in FIG. 3. The constriction 79 forms an inner diameter that is less than an outer diameter of the distal base member 40, as shown in FIG. 3. Accordingly, the distal base member 40 cannot be advanced through the distal end 74 of the catheter 70. When the spring member 50 of the first tacking device 20a is in the relaxed state shown in FIG. 3, the tissue engaging member 60 may extend partially into the constriction 79, but preferably does not extend beyond the distal end 74 of the catheter 70 to reduce the likelihood of inadvertent piercing.

Referring to FIG. 4, in a next step, the stylet 90 is advanced distally, relative to the catheter 70 and the outer sheath 80, to cause distal advancement of the second tacking device 20b and the first tacking device 20a. The stylet 90 is advanced while the outer sheath 80 continues to cover the distal end 74 of the catheter 70, thereby retaining the constriction 79. As the first tacking device 20a is advanced distally, the distal base member 40 of the first tacking device 20a is retained by the constriction 79. However, the proximal base member 30, the main body 21 and the tissue engaging member 60 of the first tacking device 20a are advanced distally relative to the constriction 79, and the spring member 50 becomes compressed between the proximal and distal base members 30 and 40, as depicted in FIG. 4. At this time, the tissue engaging member 60 is advanced distally beyond the catheter 70 and the outer sheath 80 and may pierce through one or more tissue or graft segments. In the ventral hernia example of FIG. 6, the tissue engaging member 60 may pierce through the graft member 110 and at least some of the underlying tissue 104 surrounding the perforation 105 when in the deployment configuration shown in FIG. 4.

Further, when in the deployment configuration shown in FIG. 4, the spacing L₃ shown in FIG. 2 above therefore is formed between the distal surface 44 of the distal base member 40 and the proximal edge 62 of the tissue engaging member 60. The length of the spacing L₃ may be varied based on the amount of distal advancement of the stylet 90 and corresponding compression of the spring member 50. The length of the spacing L₃ is sufficient to capture a portion of the tissue 104 and the graft member 110 between the distal surface 44 of the distal base member 40 and the proximal edge 62 of the tissue engaging member 60.

Referring now to FIG. 5, in a next step, the outer sheath 80 is proximally retracted with respect to the catheter 70, such that the distal end 84 of the outer sheath 80 is positioned proximal to the wedge member 92. At this time, the wedge member 92 is no longer radially constrained and may move in a radially outward direction, as shown in FIG. 5. The flexible distal region 75 also may move radially outward and the constriction 79 may be removed, as depicted in FIG. 5. In this configuration, an inner diameter at the distal end 74 of the catheter 70 is equal to or greater than the outer diameter of the first tacking device 20a. Therefore, the first tacking device 20a may be ejected from the distal end 74 of the catheter 70. The first tacking device 20a may be ejected either by holding the stylet 90 steady while proximally retracting the outer sheath 80 and the catheter 70 in tandem, or alternatively, by distally advancing the stylet 90 while holding the outer sheath 80 and the catheter 70 steady. After ejection from the catheter 70, the first tacking device 20a is deployed as shown in FIG. 6. The second tacking device 20b then is positioned for deployment near the distal end 74 of the catheter 70.

After deployment of the first tacking device 20a, but before deployment of the second tacking device 20b, the outer sheath 80 may be distally advanced with respect to the catheter 70, thereby urging the wedge member 92 in a radially inward direction and causing the flexible distal region 75 to move radially inward and form the constriction 79, as shown in FIG. 3 above. Subsequently, the same sequence of deployment for the first tacking device 20a, as explained with respect to FIGS. 3-5, may be used to deploy the second tacking device 20b at a second location around the perimeter of the perforation 105, as shown in FIG. 6. In this manner, any number of tacking devices may be sequentially loaded into the lumen 78 of the catheter 70 and deployed, one at a time, to at least partially surround the perforation 105.

The first and second tacking devices 20a and 20b apply a compressive force to hold the graft member 110 to the tissue 104, thereby providing a fluid tight seal around the perforation 105. In particular, the spring members 50 of the first and second tacking devices 20a and 20b are biased towards the relaxed state, shown in FIG. 1 above, and therefore the distal base member 40 is biased to securely engage a proximal surface of the graft member 110.

Advantageously, the provision of the spring member 50 facilitates a coupling of the graft member 110 to the tissue 104, regardless of a thickness t₁ of the tissue 104 and a thickness t₂ of the graft member 110. Since the spring member 50 is biased to the relaxed state of FIG. 1, it can accommodate any combined thickness **t₁ + t₂** of the tissue 104 and the graft member 110, so long as the spacing L₃ (see FIG. 2) is greater than a combined segment desired to be captured. It should be noted that when the tacking devices 20a and 20b are deployed, the biasing of the spring members 50 allows the distal base member 40 to accommodate localized variations in thickness of the tissue 104 and/or the graft member 110, without resulting in leakage. Moreover, since the distal end 54 of the spring member 50 is biased to be disposed in substantially close proximity to the tissue engaging member 60, as shown in FIG. 1 above, tissue segments of varying thicknesses, no matter how thin, may be captured between the distal surface 44 of the distal base member 40 and the tissue engaging member 60 when the spring member 50 is biased towards the relaxed state.

It should be noted that the tissue engaging member 60 may be deployed entirely within the tissue 104, as depicted in FIG. 6, or alternatively may be deployed substantially distal to the tissue 104 while abutting or piercing through a distal edge of the tissue 104. In the latter embodiment, the spacing **L₃** (see FIG. 2) between the distal surface 44 of the distal base member 40 and the proximal edge 62 of the tissue engaging member 60, when the spring member 50 is in a compressed state, will be larger than the combined thickness **t₁ + t₂** of the tissue 104 and the graft member 110. However, if the tissue engaging member 60 is deployed entirely within the tissue 104, the spacing **L₃** may be greater than, equal to, ur less than the combined thickness **t₁ + t₂** of the tissue 104 and the graft member 110, so long as the spacing L₃ permits deployment of the distal base member 40 proximal to the graft member 110.

It should be noted that the distal base member 40 optionally may be omitted. In this case, substantially identical method steps may be used to deploy the tacking device 20, however, the distal end 54 of the spring member 50 would be configured to be retained by the constriction 79 of the catheter 70, and further configured to directly apply a compressive force upon the graft member 110.

The graft member 110 may comprise any suitable material for covering the perforation 75 and substantially or entirely inhibiting the protrusion of abdominal matter. In one embodiment, the graft member 110 may comprise small intestinal submucosa (SIS), such as SURGISIS^{®} BIODESIGN™ Soft Tissue Graft, available from Cook Biotech, Inc., West Lafayette, Indiana, which provides smart tissue remodeling through its three-dimensional extracellular matrix (ECM) that is colonized by host tissue cells and blood vessels, and provides a scaffold for connective and epithelial tissue growth and differentiation along with the ECM components. Preferably, the graft member 110 would be a one to four layer lyophilized soft tissue graft made from any number of tissue engineered products. Reconstituted or naturally-derived collagenous materials can be used, and such materials that are at least bioresorbable will provide an advantage, with materials that are bioremodelable and promote cellular invasion and ingrowth providing particular advantage. Suitable bioremodelable materials can be provided by collagenous ECMs possessing biotropic properties, including in certain forms angiogenic collagenous extracellular matrix materials. For example, suitable collagenous materials include ECMs such as submucosa, renal capsule membrane, dermal collagen, dura mater, pericardium, fascia lata, serosa, peritoneum or basement membrane layers, including liver basement membrane. Suitable submucosa materials for these purposes include, for instance, intestinal submucosa, including small intestinal submucosa, stomach submucosa, urinary bladder submucosa, and uterine submucosa. The graft member 110 may also comprise a composite of a biomaterial and a biodegradeable polymer. Additional details may be found in U.S. Patent No. 6,206,931 to Cook et al., the disclosure of which is incorporated herein by reference in its entirety.

While FIG. 6 has illustrated the use of one or more tacking device 20 for covering a perforation 105 formed in the ventral abdominal wall, the tacking devices disclosed herein may be useful in many other procedures. Solely by way of example, one or more tacking devices 20 may be used to treat perforations in a visceral wall, such as the stomach wall. In such cases, a suitable insertion device, such as an endoscope, may be advanced through a bodily lumen such as the alimentary canal to a position proximate the target location. One or more components may be advanced through a working lumen of the endoscope. To close the perforation, the graft member 110 may cover the perforation and may be secured in a position overlapping the perforation using the one or more of the tacking devices 20, which may be deployed using the techniques described hereinabove. In yet further applications within the scope of the present embodiments, the tacking device 20 may be used to secure a graft member to tissue for reconstructing local tissue, and the like.

Further, the tacking device 20 need not be used for coupling a graft member to tissue. For example, the tacking devices 20 may be used in an anastomosis procedure. In order to create an anastomosis, for example, multiple tacking devices 20 may be deployed in a circular manner to couple a proximal vessel, duct or organ to a distal vessel, duct or organ. In such cases, a suitable insertion device, such as an endoscope, may be advanced through a bodily lumen such as the alimentary canal to a position proximate the target location. One or more components, such as the outer sheath 80 and the catheter 70 housing the tacking devices 20, may be advanced through a working lumen of the endoscope, and under suitable visualization, multiple tacking devices then may be delivered at one time. Then, a hole may be punched through the middle of the deployed tacking devices to create a flow path between the proximal and distal vessels/ducts/organs. It will be apparent that still further applications of the tacking devices 20 are possible, and the tacking devices may be delivered using an open technique, laparoscopic technique or via an endoscope.

Referring to FIG. 7, and as noted above, the flexible distal region 75 of the catheter 70 may be selectively moved in a radially inward and outward direction by providing a plurality of slits 77 formed in the flexible distal region 75. In the embodiment shown, four slits 77 are formed in the distal end 74 of the catheter 70 and extend in tapered manner in a distal to proximal direction. The four slits 77 may be radially spaced apart around the circumference of the catheter 70. One or more of the wedge members 92 may be attached to the flexible distal region 75 at one or more locations between the slits 77. While four illustrative tapered slits 77 are shown in FIG. 7, it will be appreciated that greater or fewer slits may be employed, and they may comprise different shapes and configurations than depicted.

Referring now to FIGS. 8-9, the tacking device 20 is deployed in the same manner as FIGS. 3-5, with the main exception that one or more alternative wedge members 92' are disposed internal to the catheter 70. Preferably, the alternative wedge members 92' comprise a triangular shape and are attached to an inner surface of the catheter 70 along the flexible distal region 75. When the outer sheath 80 is distally advanced to cover the distal end 74 of the catheter 70, the wedge member 92' moves radially inward to form the constriction 79, as shown in FIG. 7. At this time, the spring member 50 of the tacking device 20 may be compressed by distal advancement of the stylet 90, as explained in FIG. 4 above.

When it becomes desirable to release the tacking device 20, the outer sheath 80 may be proximally retracted with respect to the catheter 70 to a location proximal to the wedge member 92'. At this time, the wedge member 92' is no longer radially constrained and may move in a radially outward direction to form a substantially flush extension to the catheter 70, while the flexible distal region 75 moves radially outward, as shown in FIG. 9. At this time, the constriction 79 is removed and the tacking device 20 may be ejected from the distal end 74 of the catheter 70.

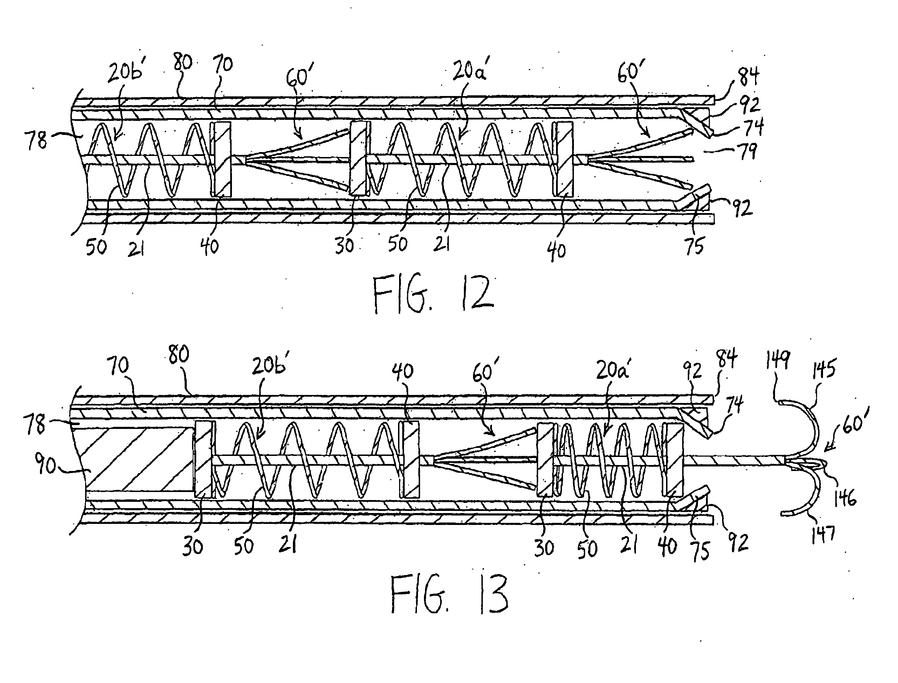

Referring now to FIGS. 10-13, an alternative embodiment of a tacking device is shown. The alternative tacking device 20' is substantially identical to the tacking device 20 of FIG. 1, with the main exception that at least one tissue engaging member 60' comprises a plurality of distal deployable members 145-147, each having expanded and contracted states. In the expanded states, the distal deployable members 145-147 may comprise a hook-shaped configuration, as shown in FIGS. 10-11 and described further below, while in the contracted states, the distal deployable members 145-147 may comprise a substantially flat profile suitable for delivery via the catheter 70, as depicted in FIG. 12 below.

The distal deployable members 145-147 extend distally from the distal end 24 of the main body 21, as shown in FIG. 10. The distal deployable members 145-147 each may be integrally formed with the main body 21 or formed separately and coupled to the main body 21. In the latter embodiment, a recess may be formed in the distal end 24 of the main body 21, and proximal regions of the three distal deployable members 145-147 may be secured within the recess of the main body 21 using an adhesive, frictional fit, mechanical device or other suitable mechanism. Alternatively, the recess may be omitted and the distal deployable members 145-147 may be coupled or adhered to an exterior surface of the main body 21 near the distal end 24.

While three total distal deployable members 145-147 are depicted, it will be apparent that greater or fewer deployable members may be employed. Moreover, the distal deployable members 145-147 may comprise any shape suitable for engaging, penetrating and/or abutting tissue, and need not necessarily assume the expanded shape depicted in FIGS. 10-11.

In one embodiment, each of the distal deployable members 145-147 comprises a curvature of about 90 to about 360 degrees in the expanded state, and more preferably about 180 degrees, as shown in FIG. 10. Where the distal deployable members 145-147 "retroflex" and comprises a curvature of about 180 degrees, end regions 149 of the distal deployable members 145-147 are oriented substantially parallel to the main body 21. Moreover, the end regions 149 may be radially spaced apart from one another in the expanded state, as shown in FIG. 10. In this configuration, the end regions 149 may be well-suited for engaging, grasping, piercing and/or abutting tissue. In the embodiments depicted herein, the end regions 149 comprise blunt tips, but alternatively may comprise sharpened tips to facilitate piercing of tissue.

The distal deployable members 145-147 may comprise a shape-memory material, such as a nickel-titanium alloy (nitinol). If a shape-memory material such as nitinol is employed, the distal deployable members 145-147 may be manufactured such that they can assume the preconfigured expanded state shown in FIG. 10 upon application of a certain cold or hot medium. More specifically, a shape-memory material may undergo a substantially reversible phase transformation that allows it to "remember" and return to a previous shape or configuration. For example, in the case of nitinol, a transformation between an austenitic phase and a martensitic phase may occur by cooling and/or heating (shape memory effect) or by isothermally applying and/or removing stress (superelastic effect). Austenite is characteristically the stronger phase and martensite is the more easily deformable phase.

In an example of the shape-memory effect, a nickel-titanium alloy having an initial configuration in the austenitic phase may be cooled below a transformation temperature (Mr) to the martensitic phase and then deformed to a second configuration. Upon heating to another transformation temperature (A_{f}), the material may spontaneously return to its initial, predetermined configuration, as shown in FIG. 10. Generally, the memory effect is one-way, which means that the spontaneous change from one configuration to another occurs only upon heating. However, it is possible to obtain a two-way shape memory effect, in which a shape memory material spontaneously changes shape upon cooling as well as upon heating.

Alternatively, the distal deployable members 145-147 may be made from other metals and alloys that are biased, such that they may be restrained by the catheter 70 prior to deployment, but are inclined to return to their relaxed, expanded configuration upon deployment. Solely by way of example, the distal deployable members 145-147 may comprise other materials such as stainless steel, cobalt-chrome alloys, amorphous metals, tantalum, platinum, gold and titanium. The distal deployable members 145-147 also may be made from non-metallic materials, such as thermoplastics and other polymers. As noted above, the distal deployable members 145-147 may comprise any shape suitable for engaging, penetrating and/or abutting tissue, for purposes explained further below, and need not necessarily assume the curved shape depicted in FIG. 10.

The tacking device 20' preferably comprises the spring member 50 described in FIGS. 1-2 above, which has relaxed and expanded states. In the relaxed state of FIG. 10, the spring member 50 is longitudinally expanded and the distal end 54 of the spring member 50 may be disposed in substantially close proximity to the tissue engaging member 60'. If the optional distal base member 40 is used, the distal surface 44 in an abutting relationship with, the distal deployable members 145-147 when the spring member 50 is in the relaxed state and the deployable members 145-147 are in the expanded states. Accordingly, one or more segments of tissue or graft material having varying thicknesses, no matter how thin, may be captured between the distal surface 44 of the distal base member 40 and the tissue engaging member 60' when the spring member 50 is biased towards the relaxed state.

In FIG. 11, the spring member 50 is in the compressed state, generally described in FIG. 2 above. In the compressed state, a spacing **L₄** is formed between the distal surface 44 of the distal base member 40 and the end region 149 of the distal deployable members 145-147. In this state, one or more segments of tissue or graft material may be positioned between the distal base member 40 and the distal deployable members 145-147.

Referring now to FIGS. 12-13, one or more tacking devices 20' may be delivered to a target site in a patient's anatomy using the catheter 70 and the outer sheath 80 described above. In FIG. 12, first and second tacking devices 20a' and 20b' are shown in the contracted states whereby the distal deployable members 145-147 may comprise a substantially longitudinally-oriented profile, i.e., oriented along a longitudinal axis of the catheter 70.

The first and second tacking devices 20a' and 20b' may be loaded sequentially such that the first tacking device 20a' is loaded distal to the second tacking device 20b' within the lumen 78 of the catheter 70, as shown in FIG. 12. The stylet 90 may be positioned in the lumen 78 at a location proximal to the second tacking device 20b'.

The outer sheath 80 is positioned over the catheter 70 and the wedge member 92 to form the constriction 79, as shown in FIG. 12 and explained above. When the first tacking device 20a' is loaded within the lumen 78, the distal deployable members 145-147 may extend partially into the constriction 79, as shown in FIG. 12, but preferably does not extend beyond the distal end 74 of the catheter 70 to reduce the likelihood of inadvertent piercing and/or inadvertent self-expansion of the distal deployable members 145-147.

Referring to FIG. 13, in a next step, the stylet 90 is advanced distally to cause distal advancement of the second tacking device 20b' and the first tacking device 20a'. In one technique, in order to facilitate distal advancement of the distal deployable members 145-147 through the constriction 79, the outer sheath 80 may be temporarily retracted proximal to the wedge member 92, thereby providing a substantially flush inner lumen and facilitating advancement of the end regions 149 of the distal deployable members 145-147 beyond the catheter 70. Once the end regions 149 have been advanced distally beyond the distal end 74 of the catheter 70, the outer sheath 80 preferably is advanced distally to urge the wedge member 92 radially inward to form the constriction 79.

The stylet 90 then is further advanced distally such that the distal base member 40 of the first tacking device 20a' is retained by the constriction 79. The proximal base member 30, main body 21 and the distal deployable members 145-147 of the first tacking device 20a' are advanced distally relative to the constriction 79, and the spring member 50 becomes compressed between the proximal and distal base members 30 and 40, as depicted in FIG. 13. At this time, the distal deployable members 145-147 arc advanced distally beyond the catheter 70 and may pierce through a tissue segment. In the ventral hernia example of FIG. 6, the distal deployable members 145-147 would pierce through the graft member 110 and at least some of the underlying tissue 104 surrounding the perforation 105.

The spacing **L₄,** shown in FIG. 11 above, therefore is formed between the distal surface 44 of the distal base member 40 and the end regions 149 of the distal deployable members 145-147. The length of the spacing **L₄** may be varied based on the amount of distal advancement of the stylet 90 and corresponding compression of the spring member 50. The length of the spacing L₄ is sufficient to capture a portion of the tissue 104 and the graft member 110 between the distal surface 44 of the distal base member 40 and the end regions 149 of the distal deployable members 145-147.

The remainder of the deployment of the first and second tacking devices 20a' and 20b' preferably is performed in accordance with the techniques described above regarding the first and second tacking devices 20a and 20b. In particular, the outer sheath 80 may be proximally retracted beyond the wedge member 92, allowing the flexible distal region 75 and the wedge member 92 to move radially outward and removing the constriction 79, as depicted in FIG. 5 above. At this time, the first tacking device 20a' may be ejected from the distal end 74 of the catheter 70. The second tacking device 20b' then is positioned for deployment near the distal end 74 of the catheter 70 and deployed in a similar manner, as explained above.

Like the first and second tacking devices 20a and 20b, the first and second tacking devices 20a' and 20b' apply a compressive force to hold the graft member 110 to the tissue 104, thereby providing a fluid tight seal around the perforation 105. Advantageously, the provision of the spring member 50 facilitates a coupling of the graft member 110 to the tissue 104, regardless of a thickness **t₁** of the tissue 104 and a thickness t₂ of the graft member 110. Since the spring member 50 is biased to the relaxed state of FIG. 10, it can accommodate any combined thickness **t₁ + t₂** of the tissue 104 and the graft member 110, so long as the spacing L₄ (see FIG. 11) is greater than a combined segment desired to be captured. It should be noted that when the tacking devices 20a' and 20b' are deployed, the biasing of the spring members 50 allows the distal base member 40 to accommodate localized variations in thickness of the tissue 104 and/or the graft member 110 without resulting in leakage.

In further alternative embodiments, the apparatus and methods described herein may be used for engaging a layer of material, and are not restricted to methods for treatment of a human or animal body by surgery or therapy. For example, the tacking device with the spring member may be delivered in the relaxed state wherein the spring member is biased to extend distally towards the at least one engaging member. A distal end of the spring member is adapted to be disposed in substantially close proximity to the at least one engaging member in the relaxed state. A compressive force is applied to the spring member to cause the spring member to assume a compressed state in which the distal end of the spring member is spaced further apart from the at least one engaging member. The engaging member is advanced to engage a layer of material when the spring member is in the compressed state, wherein at least one material layer of varying thickness is adapted to be captured between the distal end of the spring member and the at least one engaging member. The compressive force is then removed to allow the spring member to return towards the relaxed state and apply a compressive force upon the layer of material, as generally described above.

While various embodiments of the invention have been described, the invention is not to be restricted except in light of the attached claims. Moreover, the advantages described herein are not necessarily the only advantages of the invention and it is not necessarily expected that every embodiment of the invention will achieve all of the advantages described.

## Claims

1. A tacking device (20) for engaging tissue, the tacking device comprising:
a main body (21) having proximal (22) and distal (24) ends;
a proximal base member (30) disposed at the proximal end of the main body;
at least one tissue engaging member (60) disposed at the distal end of the main body;
a spring member (50) having proximal (52) and distal (54) ends, wherein the spring member is disposed to surround the main body, and wherein the proximal end of the spring member contacts the proximal base member, and
wherein the spring member has a relaxed state in which it is biased to extend distally towards the at least one tissue engaging member, wherein the distal end of the spring member abuts the at least one tissue engaging member in the relaxed state, and
wherein the spring member has a compressed state in which the distal end of the spring member is spaced further from the at least one tissue engaging member than in the relaxed state, such that in use of the tacking device at least one tissue segment may be captured between the distal end of the spring member and the at least one tissue engaging member.

2. A tacking device (20) for engaging tissue, the tacking device comprising:
a main body (21) having proximal (22) and distal (24) ends;
a proximal base member (30) disposed at the proximal end of the main body;
at least one tissue engaging member (60) disposed at the distal end of the main body;
a spring member (50) having proximal (52) and distal (54) ends, wherein the spring member is disposed to surround the main body, and wherein the proximal end of the spring member contacts the proximal base member, and
distal base member (40) having an aperture (47) configured to permit movement of the distal base member relative to the main body, wherein the distal end of the spring member contacts a proximal surface (42) of the distal base member, and wherein a distal surface (44) of the distal base member is adapted to engage tissue,
wherein the spring member has a relaxed state in which it is biased to extend distally towards the at least one tissue engaging member, wherein the distal surface of the distal base member abuts the at least one tissue engaging member in the relaxed state, and
wherein the spring member has a compressed state in which the distal end of the spring member is spaced further from the at least one tissue engaging member than in the relaxed state, such that in use of the tacking device at least one tissue segment may be captured between the distal surface of the distal base member and the at least one tissue engaging member.

3. The tacking device of claim 1 or 2, wherein the at least one tissue engaging member comprises a single member forming a sharpened hook-shaped tip (60)

4. The tacking device of claim 2, wherein the at least one tissue engaging member (20') comprises a plurality of distal deployable members (145, 146, 147) having contracted and expanded states.

5. The tacking device of claim 3, wherein the distal deployable members comprise substantially flat configurations in the contracted state and further comprises hook-shaped configurations in the expanded state.

## Patentansprüche

1. Heftvorrichtung (20) zum Eingriff in Gewebe, worin die Heftvorrichtung Folgendes umfasst:
einen Hauptkörper (21) mit proximalen (22) und distalen (24) Enden;
ein proximales Basiselement (30), das am proximalen Ende des Hauptkörpers angeordnet ist,
zumindest ein Gewebeeingriffselement (60), das am distalen Ende des Hauptkörpers angeordnet ist;
ein Federelement (50) mit proximalen (52) und distalen (54) Enden, worin das Federelement so angeordnet ist, dass es den Hauptkörper umgibt, und worin das proximale Ende des Federelements mit dem proximalen Basiselement in Berührung steht, und
worin das Federelement einen entspannten Zustand aufweist, in dem es vorgespannt ist, damit es sich distal zu dem zumindest einen Gewebeeingriffselement erstreckt, worin das distale Ende des Federelements im entspannten Zustand an das zumindest eine Gewebeeingriffselement anstößt, und
worin das Federelement einen zusammengedrückten Zustand aufweist, in dem das distale Ende des Federelements weiter von dem zumindest einen Gewebeeingriffselement beabstandet ist als im entspannten Zustand, so dass im Gebrauch der Heftvorrichtung zumindest ein Gewebesegment zwischen dem distalen Ende des Federelements und dem zumindest einen Gewebeeingriffselement erfasst werden kann.

2. Heftvorrichtung (20) zum Eingriff in Gewebe, worin die Heftvorrichtung Folgendes umfasst:
einen Hauptkörper (21) mit proximalen (22) und distalen (24) Enden;
ein proximales Basiselement (30), das am proximalen Ende des Hauptkörpers angeordnet ist,
zumindest ein Gewebeeingriffselement (60), das am distalen Ende des Hauptkörpers angeordnet ist;
ein Federelement (50) mit proximalen (52) und distalen (54) Enden, worin das Federelement so angeordnet ist, dass es den Hauptkörper umgibt, und worin das proximale Ende des Federelements mit dem proximalen Basiselement in Berührung steht, und
ein distales Basiselement (40) mit einer Öffnung (47), die ausgelegt ist, eine Bewegung des distalen Basiselements bezüglich des Hauptkörpers zu gestatten, worin das distale Ende des Federelements eine proximale Fläche (42) des distalen Basiselements berührt und worin eine distale Fläche (44) des distalen Basiselements zum Eingriff in Gewebe ausgelegt ist,
worin das Federelement einen entspannten Zustand aufweist, in dem es vorgespannt ist, damit es sich distal zu dem zumindest einen Gewebeeingriffselement erstreckt, worin die distale Fläche des distalen Basiselements im entspannten Zustand an das zumindest eine Gewebeeingriffselement anstößt, und
worin das Federelement einen zusammengedrückten Zustand aufweist, in dem das distale Ende des Federelements weiter von dem zumindest einen Gewebeeingriffselement beabstandet ist als im entspannten Zustand, so dass im Gebrauch der Heftvorrichtung zumindest ein Gewebesegment zwischen der distalen Fläche des distalen Basiselements und dem zumindest einen Gewebeeingriffselement erfasst werden kann.

3. Heftvorrichtung nach Anspruch 1 oder 2, worin das zumindest eine Gewebeeingriffselement ein einzelnes Element umfasst, das eine angespitzte hakenförmige Spitze (60) formt.

4. Heftvorrichtung nach Anspruch 2, worin das zumindest eine Gewebeeingriffselement (20') eine Vielzahl von distalen einsetzbaren Elementen (145, 146, 147) umfasst, die zusammengezogene und aufgeweitete Zustände aufweisen.

5. Heftvorrichtung nach Anspruch 3, worin die distalen einsetzbaren Element im Wesentlichen flache Konfigurationen im zusammengezogenen Zustand umfassen und ferner hakenförmige Konfigurationen im aufgeweiteten Zustand umfassen.

## Revendications

1. Dispositif de maintien en place (20) pour engager un tissu, le dispositif de maintien en place comprenant:
un corps principal (21) présentant des extrémités proximale (22) et distale (24);
un élément de base proximal (30) qui est disposé à l'extrémité proximale du corps principal;
au moins un élément d'engagement de tissu (60) qui est disposé à l'extrémité distale du corps principal;
un élément de ressort (50) présentant des extrémités proximale (52) et distale (54), dans lequel l'élément de ressort est disposé de manière à entourer le corps principal, et dans lequel l'extrémité proximale de l'élément de ressort entre en contact avec l'élément de base proximal, et
dans lequel l'élément de ressort présente un état relâché, dans lequel il est poussé de manière à s'étendre de façon distale en direction dudit au moins un élément d'engagement de tissu, dans lequel l'extrémité distale de l'élément de ressort bute contre ledit au moins un élément d'engagement de tissu à l'état relâché; et
dans lequel l'élément de ressort présente un état comprimé, dans lequel l'extrémité distale de l'élément de ressort est espacée davantage dudit au moins un élément d'engagement de tissu que dans l'état relâché, de telle sorte que, lors de l'utilisation du dispositif de maintien en place, au moins un segment de tissu puisse être capturé entre l'extrémité distale de l'élément de ressort et ledit au moins un élément d'engagement de tissu.

2. Dispositif de maintien en place (20) pour engager un tissu, le dispositif de maintien en place comprenant:
un corps principal (21) présentant des extrémités proximale (22) et distale (24);
un élément de base proximal (30) qui est disposé à l'extrémité proximale du corps principal;
au moins un élément d'engagement de tissu (60) qui est disposé à l'extrémité distale du corps principal;
un élément de ressort (50) présentant des extrémités proximale (52) et distale (54), dans lequel l'élément de ressort est disposé de manière à entourer le corps principal, et dans lequel l'extrémité proximale de l'élément de ressort entre en contact avec l'élément de base proximal, et
un élément de base distal (40) comportant une ouverture (47) configurée de manière à permettre un déplacement de l'élément de base distal par rapport au corps principal, dans lequel l'extrémité distale de l'élément de ressort entre en contact avec une surface proximale (42) de l'élément de base distal, et dans lequel une surface distale (44) de l'élément de base distal est adaptée pour engager un tissu,
dans lequel l'élément de ressort présente un état relâché, dans lequel il est poussé de manière à s'étendre de façon distale en direction dudit au moins un élément d'engagement de tissu, dans lequel la surface distale de l'élément de base distal bute contre ledit au moins un élément d'engagement de tissu à l'état relâché; et
dans lequel l'élément de ressort présente un état comprimé, dans lequel l'extrémité distale de l'élément de ressort est espacée davantage dudit au moins un élément d'engagement de tissu que dans l'état relâché, de telle sorte que, lors de l'utilisation du dispositif de maintien en place, au moins un segment de tissu puisse être capturé entre la surface distale de l'élément de base distal et ledit au moins un élément d'engagement de tissu.

3. Dispositif de maintien en place selon la revendication 1 ou 2, dans lequel ledit au moins un élément d'engagement de tissu comprend un seul élément qui forme une pointe tranchante en forme de crochet (60).

4. Dispositif de maintien en place selon la revendication 2, dans lequel ledit au moins un élément d'engagement de tissu (20') comprend une pluralité d'éléments distaux déployables (145, 146, 147) qui présentent des états contractés et étendus.

5. Dispositif de maintien en place selon la revendication 3, dans lequel les éléments distaux déployables présentent des configurations sensiblement plates à l'état contracté, et présentent en outre des configurations en forme de crochet à l'état étendu.
